# EUROPEAN PATENT APPLICATION

(11) **EP 2 979 649 A1**
(43) Date of publication of application: **03.02.2016**
(21) Application number: 14776485.6
(22) Date of filing: 26.03.2014
(51) Int. Cl.: A61B 17/22, A61B 17/32

(54) **INTRACRANIAL VASCULAR THROMBECTOMY DEVICE AND THROMBECTOMY APPARATUS**

(30) Priority: 26.03.2013 CN 201310100143
(71) Applicant: Shanghai MicroPort Medical (Group) Co., Ltd., Shanghai 201203 (CN)
(72) Inventor: XU, Qingshun, Shanghai 201203 (CN); NI, Zunzhang, Shanghai 201203 (CN); XIE, Zhiyong, Shanghai 201203 (CN); JIN, Qiaorong, Shanghai 201203 (CN); JI, Jie, Shanghai 201203 (CN); LIU, Cong, Shanghai 201203 (CN); CHANG, Mengqi, Shanghai 201203 (CN); LUO, Qiyi, Shanghai 201203 (CN)
(74) Representative: Tischner, Oliver
(86) International application number: PCT/CN2014/074070
(87) International publication number: WO 2014/154137

(57) **Abstract**

A thrombus removal means (10) and a thrombus removal device (1) particularly suited to the removal of a thrombus from an intracranial vessel. The thrombus removal means (10) is a mesh structure defining a lumen and is switchable between a contracted configuration and an expanded configuration, the thrombus removal means (10) has inwardly and/or outwardly projecting features. The thrombus removal means (10) can hold a captured thrombus firmly during the retrieval, effectively trap plaques and clot fragments with an improved stiffness and with no possibility for them to dislodge therefrom, and can addresses the issue of distal re-embolization caused by the vessel wall plaques or clot fragments dislodging from the conventional thrombus removal devices by presenting an improved thrombus removal system with the additional benefit of a small profile and high flexibility.

## Description

### TECHNICAL FIELD

The present invention relates to medical instruments and, in particular, to a medical instrument for vascular intervention and, more particularly, to a means for removing a thrombus from an intracranial vessel. The invention is also directed to a thrombus removal device incorporating the means for removing the thrombus.

### BACKGROUND

Intravascular thrombosis is a high-incidence disease frequently encountered in clinical practice, and intracranial thrombosis has the most serious consequences and can lead to cerebral infarction. Intracranial thrombosis is known to have high incidence, morbidity, mortality and recurrence rate, and is ranked as a leading cause of disability and death among middle-aged and elderly people. With the increasing elderly population and living standards, the number of patients with cerebrovascular diseases has been increasing. Surveys reveal that, in China, three million people are initially diagnosed with cerebral thrombosis every year and acute strokes have accounted for 81.6% of all strokes, compared to the previous percentage of 55.8%, becoming the most frequent disease of the brain.

Blood flow restoration is crucial for the treatment of acute ischemic strokes. At present, the treatment of intracranial thrombosis is based essentially on lysis by thrombolytic drugs and mechanical removal.

The lysis approach involves infusing a thrombolytic agent around an occlusive thrombus in a blood vessel to instantly create there a high concentration of the agent to facilitate lysis of the thrombus and increase the likelihood of blood flow restoration. However, according to findings of the U.S. National Institute of Neurological Disorders and Stroke (NINDS), intravenous lysis should be effectuated within 3 hours since the onset, and the time window for intra-arterial lysis is only 6 hours. Such limited time windows narrow the application of this treatment only to small clots and make it unfavorable for relatively large clots. As a result, only about 3-5% of patients are suitable for the lysis approach.

Mechanical removal is to deliver a thrombus removal device to a lesion, which helps to retrieve a clot by means of a sheath. Mechanical removal may be accomplished in several ways such as, surgical resection, which allows relatively thorough removal but tends to cause serious vessel wall damage and various inflammatory complications; laser fragmentation, which requires complicated operations and strict control of used laser energy, because insufficient energy is not effective and excessively high energy can cause vessel damage, and also tends to cause various inflammatory complications; use of capture devices, which allows simple operations and minimal vessel wall damage but often fails to engage a thrombus; and use of capture nets, which also allows simple operations but is unusable in intracranial vessels because the capture nets are typically too bulky.

Chinese Utility Model Application No. 03210077.9 disclosed an ellipsoidal distal protection device, comprising a stented ellipsoidal main body tapered at the both ends and a porous polymer membrane covering an upper portion of the stent. The membrane allows the passage of normal blood flow and traps solid particles such as dislodging plaques or emboli, thereby protecting downstream vessel from being occluded. Chinese Utility Model Application No. 200620164685.4 disclosed a thrombus removal device for use in lower limb arteries, consisting of an umbrella portion, a main shaft, a pusher guide wire, an inner catheter, an outer sheath and a core in the outer sheath. The umbrella portion is made up of shape memory alloy wires, two long struts and one short strut, and is angularly soldered to the main shaft. Additionally, a mesh structure for trapping emboli is disposed around the umbrella portion. U.S. Patent Publication Nos. 2007/0005103, 2008/0243170 and 2011/0098738 disclosed emboli capturing devices with the similar structures, each comprising a stent and a filter mesh or membrane for trapping emboli that is attached to the stent. All of these devices, however, are too bulky to be delivered to an intracranial vessel.

Chinese Patent Application No. 01114889.6 disclosed a reusable temporary emboli filter which can be used in intracranial vessels. The emboli filter is comprised of a filter body, a guide tip, a guide pole, an interconnection and an outer sheath. The filter body is braided from shape memory alloy wires. U.S. Patent Publication No. 2011/0160763 disclosed a thrombus removal device also useable in intracranial vessels. This thrombus removal device includes a scaffold for capturing a thrombus, which can be braided from shape memory alloy wires. However, these thrombus removal devices are both structurally complicated and lack of stiffness for holding a captured thrombus or embolus during the retrieval process. As a result, it is possible for the captured thrombus or embolus to dislodge during the retrieval process.

Furthermore, use of the conventional thrombus removal devices tends to cause distal embolization due to possible dislodgement of plaques from the vessel wall or formation of clot fragments during the use. It has been reported that fragmented pieces and pellets of blood clots travelling toward distal vessels, which expose the patients to an increased risk of vascular reocclusion, are witnessed in about 70% of endovascular thrombus removal procedures.

Therefore, there is a need for an improved thrombus removal device, which 1) entails a simple and convenient thrombus removal process; 2) provides a high flexibility, high capturing ability and high radial force while causing no vascular injury; and 3) more firmly holds the captured thrombus with less likelihood of dislodgement during the retrieval.

### SUMMARY OF THE INVENTION

In view of this, the present invention addresses the issue of distal re-embolization caused by the vessel wall plaques or clot fragments dislodging from the conventional thrombus removal devices by presenting an improved thrombus removal system with the additional benefit of a small profile and high flexibility.

According to one aspect, the present invention provides a thrombus removal means, which is particularly suitable for removing a thrombus from an intracranial vessel. The thrombus removal means is a mesh structure and is switchable between a contracted configuration and an expanded configuration. The thrombus removal means is characterized in being configured with inwardly and/or outwardly projecting features. The features can be formed either integrally with the means, or discretely therefrom and attached thereto by a suitable means. This enables a scattered distribution of capture forces and can thus prevent a captured thrombus from dislodging during the retrieval.

According to preferred embodiments of the present invention, the thrombus removal means is formed by laser cutting a metal tube and by performing a heat treatment. It is a matter of course that the thrombus removal means may also be formed by laser cutting a sheet formed of a shape memory metal or a high-elasticity polymer, folding the cut sheet and performing a heat treatment. The thrombus removal means formed in this way has good performance in terms of flexibility and vessel wall adherability. The metal may be nitinol, a cobalt-nickel alloy, stainless steel or the like.

Preferably, the thrombus removal means assumes a substantially cylindrical shape when in the expanded configuration. This imparts sufficient radial force to the thrombus removal means so that, after the means is released into the blood from an outer sheath of a thrombus removal device, it can adhere to the vessel wall in a desirable manner.

Preferably, the thrombus removal means has a substantially oval opening at its proximal end. This allows the means to be easily crimped into the outer sheath of the device while ensuring a sufficient area of the means for thrombus capture.

Preferably, the thrombus removal means is closed at its distal end. This effectively prevents a small thrombus that has been captured by the means from dislodging from the distal end during the retrieval. The closure may be accomplished by a soldering joint, a metal sleeve or a metal wire coil. The sleeve or coil may be formed of platinum, gold, tungsten, platinum-tungsten, platinum-iridium, etc. and may function as a radiopaque marker.

Preferably, center points of longitudinally adjacent cells of the thrombus removal means are spaced by distances decreasing from the proximal end to the distal end so that the cells around the distal end are denser than those around the proximal end. In embodiments of the invention, the distances range from 3 mm to 15 mm. It is a matter of course that the distances may also be equal to one another.

According to another aspect, the present invention provides a thrombus removal device, comprising a guide wire, a pusher wire, an outer sheath and an inner tube movable relative to the outer sheath, wherein the guidewire is inserted through the inner tube which is in turn received in the outer sheath and is movable over the guide wire. The thrombus removal device is characterized in further comprising a thrombus removal means as defined above, wherein the thrombus removal means is attached to the pusher wire and is crimped into the inner tube together with the pusher wire after they are so assembled, and wherein the thrombus removal means is switchable between a contracted configuration and an expanded configuration upon being retracted or advanced by the pusher wire such that the thrombus removal means is in the contracted configuration when received in the inner tube and transitions to the expanded configuration after being pushed out of the inner tube. This thrombus removal device is capable of firmly holding a captured thrombus with an improved stiffness and with no possibility for the thrombus to dislodge therefrom.

Preferably, the thrombus removal means is attached to the pusher wire by a tethering connector which is positioned closely adjacent to a vessel wall when the thrombus removal means is in the expanded configuration. This significantly facilitates the capture of a large clot. The tethering connector may be implemented as a soldering joint, a metal sleeve or a metal wire coil. The sleeve or coil may be formed of platinum, gold, tungsten, platinum-tungsten, platinum-iridium, etc. and may function as a radiopaque marker.

According to a further aspect of the invention, there is provided use of a thrombus removal device for removing a thrombus from an intracranial vessel having an occlusive thrombus. In the use, the intracranial vessel may be first identified. The outer sheath may then be delivered around the thrombus, followed by inserting the guide wire through the thrombus. The inner tube may then be delivered over the guide wire until it passes through the thrombus. After the positioning, the inner tube may be retracted to cause the thrombus removal means to expand within the thrombus. After that, the vessel may be occluded with a balloon catheter, and both of the means and inner tube may be retracted within the outer sheath, which may be in turn subsequently withdrawn from the body.

The thrombus removal means and device according to the present invention can effectively trap plaques and clot fragments with an improved stiffness and with no possibility for them to dislodge therefrom. This can result in a reduction in the risk of secondary embolization. The means and device are particularly suitable for removing a thrombus from an intracranial vessel. Of course, they can also be suitably used for other vessels for this purpose. Further, the means and device are structurally simple and entails a simple thrombus removal process.

### BRIEF DESCRIPTION OF THE DRAWINGS

The features and advantages of the present invention will become readily apparent from the following detailed description of embodiments thereof, which is to be read in connection with the accompanying drawings, in which:
FIG. 1 is a schematic illustrating the overall structure of a thrombus removal device in accordance with an embodiment of the present invention;
FIG. 2 is a schematic illustration of a thrombus removal means in accordance with an embodiment of the present invention;
FIG. 3 shows an arrangement of cells of a thrombus removal means according to the present invention;
FIGs. 4 to 11 show cross-sectional views of thrombus removal means in accordance with embodiments of the present invention taken along the line A-A of FIG. 2.

### DETAILED DESCRIPTION

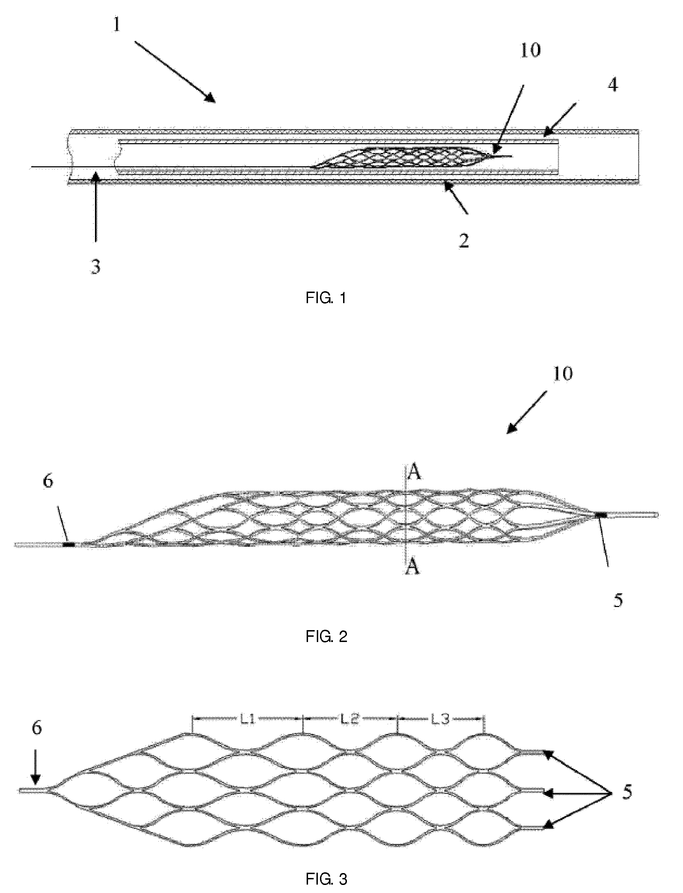

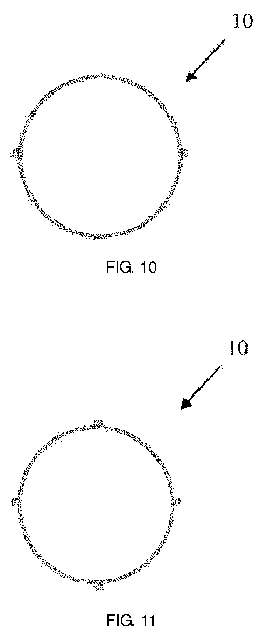

Embodiments of the present invention are described in detail blow with reference to FIGs. 1-11.

For the sake of description, the terms "proximal end" and "distal end" are used in this specification, in which, the term "proximal end" refers to an end located nearer to an operating physician, while the term "distal end" refers to an end located farther from the operating physician.

FIG. 1 shows the overall structure of a thrombus removal device 1, which includes an outer sheath 2, a pusher wire 3, an inner tube 4 and a thrombus removal means 10. The inner tube is received in the outer sheath 2 and is movable relative thereto. The thrombus removal means 10 is attached to the pusher wire 3, and after being so assembled, the pusher wire 3 and the thrombus removal means 10 are crimped together into the inner tube 4. The thrombus removal means 10 can switch between a contracted configuration and an expanded configuration upon being retracted or advanced by the pusher wire 3, such that the thrombus removal means 10 is received in the inner tube 4 when in the contracted configuration, and transitions to the expanded configuration after being pushed out of the inner tube 4. The thrombus removal device 1 may also include a guide wire (not shown) which is inserted through the inner tube 4 such that the inner tube 4 can move over the guide wire.

In addition, as shown in FIGs. 2 and 3, the thrombus removal means 10 may be provided with radiopaque markers 5 and 6 for facilitating the positioning of the thrombus removal means 10. It is to be understood that, the inner tube 4 and the outer sheath 2 may also be provided with radiopaque markers (not shown).

As shown in FIG. 2, the thrombus removal means 10 is configured as a mesh structure having a substantially cylindrical shape. In this embodiment, the thrombus removal means 10 has a length of 30-60 mm, a diameter of 2-6 mm, a wall thickness of 0.04-0.10 mm. In addition, the thrombus removal means 10 has, at its proximal end, an elliptical opening with a major diameter of 10-25 mm and a minor diameter of 2-8 mm.

Additionally, center points of longitudinally adjacent cells of the thrombus removal means 10 are spaced by distances that are equal or decrease from its proximal end to distal end, i.e., L1>L2>L3, so that the cells around the distal end are denser than those around the proximal end, as shown in FIG. 3. In this embodiment, the distances range from 3 mm to 15 mm.

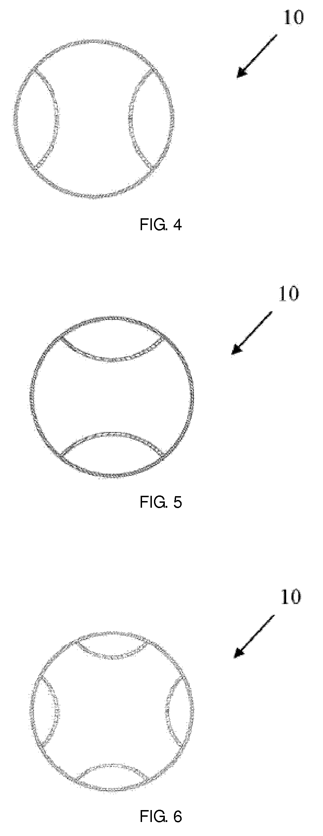

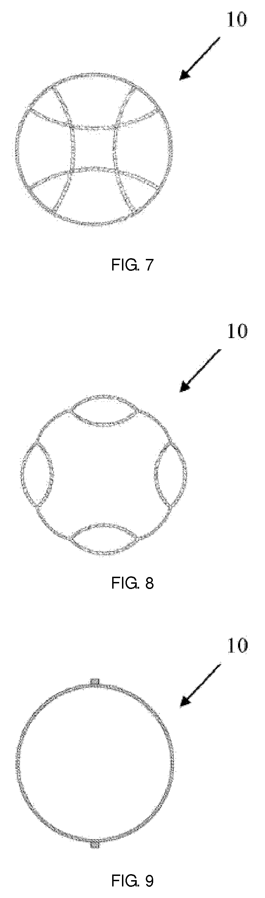

FIGs. 4 to 11 show several embodiments of the thrombus removal means of the present invention. As illustrated in FIGs. 4 to 7, the thrombus removal means may be configured with inwardly projecting features distributed symmetrically horizontally and/or vertically. In addition, the inwardly projecting features may also cross one another, as shown in FIG. 7. Alternatively, as shown in FIGs. 9 to 11, the thrombus removal means may be configured with outwardly projecting features distributed symmetrically horizontally and/or vertically. Still alternatively, as shown in FIG. 8, the thrombus removal means may be configured with both inwardly and outwardly projecting features. Of course, the thrombus removal means 10 may also be configured with inwardly and/or outwardly projecting features in other forms.

The thrombus removal means 10 may be formed by laser cutting a metal tube (e.g., a nitinol tube) and then performing a heat treatment. It is a matter of course that the thrombus removal means 10 may also be formed by laser cutting a sheet formed of a shape memory metal, folding the cut sheet and performing a heat treatment. The metal used in these formation processes may also be substituted for a high-elasticity polymer. Materials suitable to be used in these processes are well known to those skilled in the art, and therefore further description in this regard is believed unnecessary.

As shown in FIG. 2, the proximal end of the thrombus removal means 10 may be configured as an oval-shaped opening. Additionally, the thrombus removal means 10 may be closed at its distal end. The closure may be accomplished by a soldering joint, a metal sleeve or a metal wire coil. In this embodiment, the closure further functions as a radiopaque marker 5 for the distal end.

The thrombus removal means 10 may be attached to the pusher wire 3 by a tethering connector which is positioned closely adjacent to a vessel wall when the thrombus removal means is in the expanded configuration. This can significantly facilitate the capture of a large clot. In this embodiment, as shown in FIG. 2, the tethering connector further functions as a radiopaque marker 6.

The thrombus removal device 1 according to the present invention may be used in the following manner.

In an interventional treatment, an angiographic assessment is first performed to identify an intracranial blood vessel having an occlusive thrombus. The thrombus removal device 1 is then introduced in the vessel via the femoral artery or brachial artery, with the guide wire inserted through the thrombus. The inner tube 4 is then advanced over the guide wire through the thrombus to a desired position with the aid of a radiopaque marker (not shown) on the inner tube 4, with the thrombus removal means 10 being received in the inner tube 4, i.e., in the contracted configuration. Afterward, the guide wire is retrieved, and the thrombus removal means 10 is pushed by the pusher shaft toward a distal end of the inner tube 4. The inner tube 4 is then retracted into the thrombus to deploy the thrombus removal means 10 therein to a desired position by the virtue of a radiopaque marker on the thrombus removal means 10. Because of the shape memory effect of its material or the elasticity of the high-elasticity polymer, the thrombus removal means 10 switches under the action of the blood temperature to the expanded configuration and pushes against the vessel wall. The thrombus removal means 10 is retracted by the pusher shaft 3 in a state of engaging the thrombus until the thrombus removal means 10 is received within the inner tube 4 which is then, in turn, retracted into the outer sheath 2. As a last step of the removal process, the thrombus removal device 1 is wholly retrieved from the human body.

During the use of conventional thrombus removal systems, dislodgement of plaques adhering to the vessel wall, as well as fragmentation of clots, tends to occur. The plaques and clot fragments then travel with the blood flow and may cause secondary embolization in distal vessels, thus exposing the patients to increased operative risk. According to the present invention, and its dislodgement during the retrieval is therefore effectively prevented.

While the above description is made in the context of removal of a thrombus from an intracranial vessel, it is to be understood that the thrombus removal means and device of the present invention may also be used in thrombus removal for other vessels with suitable sizes. Those skilled in the art will realize that the above description is merely exemplary. Those skilled in the art can make various changes and modifications to the present invention without departing from the spirit and scope thereof.

## Claims

1. A thrombus removal means, which is a mesh structure and is switchable between a contracted configuration and an expanded configuration, wherein the thrombus removal means has inwardly and/or outwardly projecting features.

2. The thrombus removal means of claim 1, wherein the thrombus removal means assumes a substantially cylindrical shape when in the expanded configuration.

3. The thrombus removal means of claim 2, wherein the thrombus removal means has a substantially oval opening at a proximal end thereof.

4. The thrombus removal means of any of claims 1 to 3, wherein the thrombus removal means is closed at a distal end thereof.

5. The thrombus removal means of any of claims 1 to 3, wherein center points of longitudinally adjacent cells of the thrombus removal means are spaced by distances that are equal or decrease from a proximal end to a distal end so that the cells around the distal end are denser than the cells around the proximal end.

6. The thrombus removal means of claim 5, wherein the distances range from 3 mm to 15 mm.

7. A thrombus removal device, comprising a guide wire, a pusher wire, an outer sheath and an inner tube movable relative to the outer sheath, the guide wire being inserted through the inner tube which is in turn received in the outer sheath and is movable over the guide wire, wherein the thrombus removal device further comprises a thrombus removal means as defined in any of claims 1 to 6, the thrombus removal means being attached to the pusher wire and crimped into the inner tube together with the pusher wire, the thrombus removal means being switchable between a contracted configuration and an expanded configuration upon being retracted or advanced by the pusher shaft such that the thrombus removal means is in the contracted configuration when received in the inner tube and transitions to the expanded configuration after being pushed out of the inner tube.

8. The thrombus removal device of claim 7, wherein the thrombus removal means is attached to the pusher wire by a tethering connector which is positioned closely adjacent to a vessel wall when the thrombus removal means is in the expanded configuration.

9. Use of a thrombus removal device as defined in claim 7 or 8, for removing a thrombus from an intracranial vessel.
